# EUROPEAN PATENT APPLICATION

(11) **EP 3 263 008 A1**
(43) Date of publication of application: **03.01.2018**
(21) Application number: 16888046.6
(22) Date of filing: 28.09.2016
(51) Int. Cl.: A61B 1/00

(54) **ENDOSCOPE**

(30) Priority: 25.01.2016 JP 2016011465
(71) Applicant: Olympus Corporation, Hachioji-shi, Tokyo 192-8507 (JP)
(72) Inventor: NAKADE, Sho, Hachioji-shi Tokyo 192-8507 (JP)
(74) Representative: Schicker, Silvia
(86) International application number: PCT/JP2016/078633
(87) International publication number: WO 2017/130460

(57) **Abstract**

An endoscope 1 includes: an operation lever portion 30 provided in an operation portion 3, the operation lever portion 30 including an operation shaft 32 and being capable of adjusting a bending angle of a bending portion 7 in linkage with a tilting action; a sliding body 37 disposed so as to be freely movable back and forth in an axial direction of the operation shaft 32; a flexible exterior cover 33 watertightly fixed to the operation portion 3 so as to cover an outer periphery of the sliding body 37 and the operation shaft 32, the flexible exterior cover 33 being deformable by fluctuation of an internal pressure of the operation portion 3; and a return member 38 configured to return the sliding body 37 to a side of the operation portion 3 when the internal pressure of the operation portion 3 is depressurized.

## Description

### Technical Field

The present invention relates to an endoscope, a bending portion of which is bent in linkage with a tilting operation to an operation lever.

### Background Art

Conventionally, in order to observe a part inside a subject where observation is difficult, such as inside of a living body or inside of a structure, an endoscope that can be inserted into the subject is widely utilized in a medical field or an industrial field, for example.

An insertion portion of such an endoscope is provided with a bending portion for improving insertability and observability inside a subject. The bending portion is bent by a bending operation device provided in an operation portion.

As the bending operation device, for example, as disclosed in Japanese Patent Application Laid-Open Publication No. 2004-321612, the bending operation device of a joystick type configured to bend a bending portion by a tilting operation to an operation lever is widely known.

In such a conventional joystick type bending operation device, in order to secure watertightness inside an endoscope while allowing a tilting action of a bending lever projecting to an outside of an operation portion, an outer side of the operation lever is watertightly covered with an exterior cover formed of an elastic member such as rubber.

Incidentally, for the endoscope of this kind, a leak test for determining whether or not watertightness of the inside is secured is performed before cleaning is performed after use. The leak test is generally performed by applying a positive pressure to the inside of the endoscope manually by a user or automatically by an endoscope cleaning apparatus through a ventilation portion provided in the endoscope.

However, in a case of pressurizing the inside of the endoscope including the joystick type bending operation device as described above to the positive pressure, the exterior cover covering an outer side portion of the operation lever is expanded by elastic deformation accompanying rise of an internal pressure. At the time, when the internal pressure is raised too much, an extension allowance part of the exterior cover is sometimes deformed and folded.

Therefore, it is needed to perform cleaning by an automatic endoscope cleaning apparatus which depressurizes the inside of the endoscope after a manual leak test and then does not perform a leak test, or to perform cleaning by an automatic endoscope cleaning apparatus which executes a cleaning process after completely depressurizing the inside after an automatic leak test, there are problems that it is complicated and takes time and labor and a special automatic endoscope cleaning apparatus needs to be chosen.

Then, the present invention is implemented in consideration of above-described circumstances, and an object is to provide an endoscope that prevents an exterior cover covering an outer side portion of an operation lever from being folded in a state in which a positive pressure is applied to the inside in a leak test process, does not need time and labor of a manual leak test, and does not choose a model of an automatic endoscope cleaning apparatus.

### Disclosure of Invention

### Means for Solving the Problem

An endoscope according to one aspect of the present invention includes: an operation portion provided on a proximal end side of an insertion portion including a bending portion; an operation lever portion provided in the operation portion, the operation lever portion including an operation shaft and being capable of adjusting a bending angle of the bending portion in linkage with a tilting action; a sliding body disposed so as to be freely movable back and forth in an axial direction of the operation shaft; a flexible exterior cover watertightly fixed to the operation portion so as to cover an outer periphery of the sliding body and the operation shaft, the flexible exterior cover being deformable by fluctuation of an internal pressure of the operation portion; and a return member configured to return the sliding body to a side of the operation portion when the internal pressure of the operation portion is depressurized.

### Brief Description of the Drawings

Fig. 1 is a front view illustrating an appearance configuration of an endoscope as one aspect of the present invention;
Fig. 2 is a right side view illustrating an appearance configuration of an operation portion of the endoscope as one aspect of the present invention;
Fig. 3 is a partial sectional view illustrating a configuration of a bending operation lever portion as one aspect of the present invention;
Fig. 4 is a partial sectional view illustrating the configuration of the bending operation lever portion in a state in which a positive pressure is applied during a leak test as one aspect of the present invention;
Fig. 5 is a perspective view illustrating a configuration of a bending operation lever portion of a first modification as one aspect of the present invention;
Fig. 6 is a partial sectional view illustrating a configuration of a bending operation lever portion of a second modification as one aspect of the present invention;
Fig. 7 is an exploded perspective view illustrating the configuration of the bending operation lever portion of the second modification as one aspect of the present invention;
Fig. 8 is a right side view illustrating an appearance configuration of an operation portion of an endoscope of a third modification as one aspect of the present invention;
Fig. 9 is a conceptual diagram illustrating a structure configured to lock/unlock a sliding body by a lock lever of the third modification as one aspect of the present invention; and
Fig. 10 is a perspective view illustrating a structure configured to lock/unlock a sliding body by a lock lever of a fourth modification as one aspect of the present invention.

### Best Mode for Carrying Out the Invention

Hereinafter, a preferable mode of the present invention will be described with reference to the drawings. Note that, in the respective drawings used in following description, a scale is made different for each component in order to turn the individual components to such sizes that the components can be recognized on the drawings, and the present invention is not limited only to quantities of the components, shapes of the components, ratios of the sizes of the components, and relative positional relationships of the individual components described in the drawings. In addition, in the following description, upper and lower directions in a view toward a paper surface of the drawing are sometimes described as an upper part and a lower part of the component.

First, an endoscope as one aspect of the present invention will be described below based on the drawings.

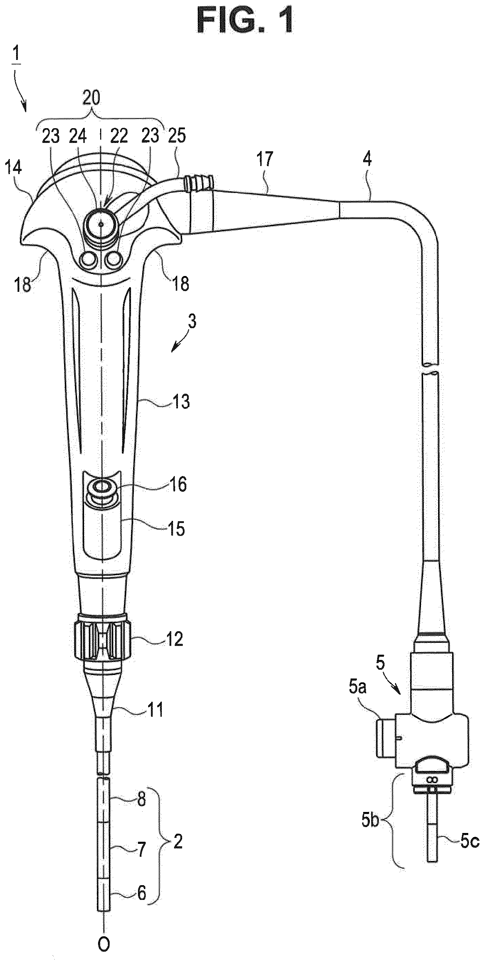

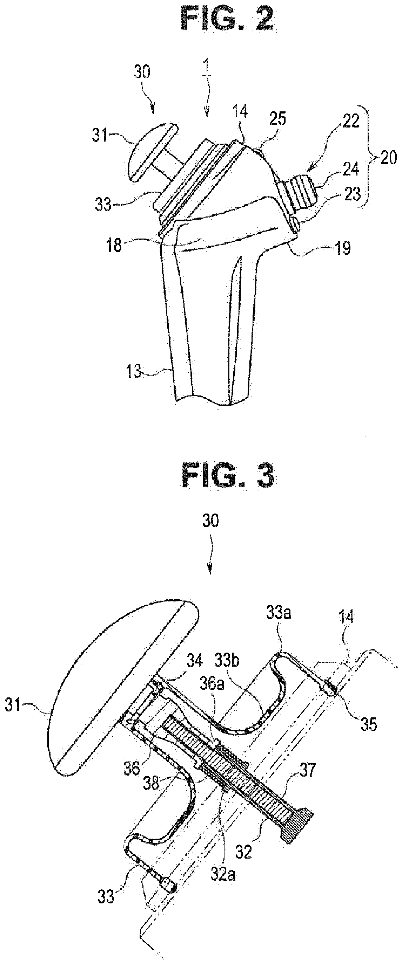

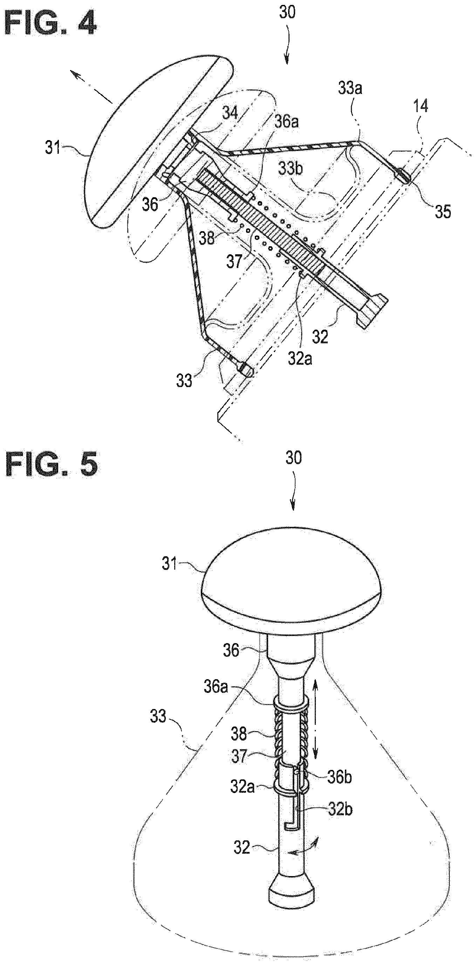

Note that Fig. 1 is a front view illustrating an appearance configuration of the endoscope, Fig. 2 is a right side view illustrating an appearance configuration of an operation portion of the endoscope, Fig. 3 is a partial sectional view illustrating a configuration of a bending operation lever portion, and Fig. 4 is a partial sectional view illustrating the configuration of the bending operation lever portion in a state in which a positive pressure is applied during a leak test.

An endoscope 1 according to an embodiment illustrated in Fig. 1 and Fig. 2 is an electronic endoscope, and is configured including an insertion portion 2 formed in an elongated tube shape, an operation portion 3 connected to a proximal end of the insertion portion 2, a universal cord 4 which is an endoscope cable extended from the operation portion 3, and an endoscope connector 5 disposed at a distal end of the universal cord 4.

The insertion portion 2 of the endoscope 1 is configured by a flexible tubular member for which a distal end portion 6, a bending portion 7 and a flexible tube portion 8 are connected in order from a distal end side.

Inside the distal end portion 6 of the insertion portion 2, though not illustrated here, an objective optical system, an image pickup unit with a built-in image sensor of CCD, CMOS or the like, an illumination optical system configured to radiate illumination light transmitted by a light guide bundle, a channel pipe configured to connect and hold a treatment instrument channel, and the like are disposed.

The bending portion 7 of the insertion portion 2 is configured so as to be actively bent in an entire circumferential direction around an insertion axis O including up-down/right-left directions, according to operation input of a user who is an operator or the like to the operation portion 3.

The flexible tube portion 8 of the insertion portion 2 is configured by a flexible tubular member that can be passively bent. Through an inside of the flexible tube portion 8, an image pickup cable, the light guide bundle, a treatment instrument insertion channel, and an air/water feeding tube are inserted (none is illustrated).

The operation portion 3 of the endoscope 1 is configured including a bend preventing portion 11 connected to the flexible tube portion 8 in a state of covering the proximal end of the flexible tube portion 8, an insertion portion rotation dial 12 capable of freely adjusting a rotating position around the insertion axis O of the insertion portion 2 provided on a proximal end side of the bend preventing portion 11, a grasping portion 13 that is connected to the proximal end side of the insertion portion rotation dial 12 and can be grasped by a hand of the user or the like, and an operation portion main body 14 connected to the proximal end side of the grasping portion 13.

Note that, in the present embodiment, a direction around the insertion axis O as a longitudinal axis in the operation portion 3 or the like is defined with the state in which the user or the like is grasping the grasping portion 13 as a reference, and specifically, front-back/right-left directions (a front surface, a back surface, left and right side faces, and the like) with the user or the like grasping the grasping portion 13 as the reference are defined in the operation portion 3.

The grasping portion 13 is formed in a bilaterally symmetrical shape to the insertion axis O, and can be grasped by the user or the like similarly by either left or right hand.

On the front surface on the distal end side of the grasping portion 13, a treatment instrument insertion portion 15 is provided. The treatment instrument insertion portion 15 is configured including a treatment instrument insertion opening 16 configured to insert various kinds of treatment instruments not illustrated.

Inside the operation portion 3, to the treatment instrument insertion opening 16, the treatment instrument insertion channel is communicated through a branching member (neither is illustrated). In addition, to/from the treatment instrument insertion portion 15, a disposable forceps plug not illustrated, which is a lid member for closing the treatment instrument insertion opening 16, for example, is freely attachable and detachable.

The operation portion main body 14 is configured by a hollow member forming a roughly partially spherical shape swollen mainly to right and left sides and front on the proximal end side of the grasping portion 13. On a front surface side of the operation portion main body 14, operation buttons 20 for executing a suction function, various kinds of optical system functions, and the like of the endoscope 1 are disposed.

The operation buttons 20 are configured including a disposable suction valve 22 for example freely detachably mounted on the operation portion main body 14, and two button switches 23 to which an arbitrary function, a release button for example, can be selectively allocated from various kinds of functions regarding the endoscope 1, for example.

Note that the suction valve 22 is configured including a suction button 24 as an operation input member, and a tube connection portion 25 to which a suction tube extended from an endoscope suction device that is an external device not illustrated is connected.

On a back surface side of the operation portion main body 14, as illustrated in Fig. 2, a bending operation lever portion 30 as bending operation means for performing a bending operation to the bending portion 7 is disposed.

From one side portion (a left side portion, for example) of the operation portion main body 14, the universal cord 4 is extended through a cable bend preventing portion 17.

Here, as illustrated in Fig. 1, left and right shapes of the operation portion main body 14 are a swollen shape bilaterally symmetrical to the insertion axis O, and on left and right side faces on the distal end side of the operation portion main body 14, a guiding recessed portion 18 configured to guide a pointing finger or the like of the user or the like grasping the grasping portion 13 to the operation buttons 20 is formed respectively.

The universal cord 4 is a composite cable through which various kinds of signal lines including the image pickup cable reaching the operation portion 3 from the side of the distal end portion 6 through the inside of the insertion portion 2 and extended from the operation portion 3 further, the light guide bundle and the air/water feeding tube into which air/water feeding fluid is made to flow (none is illustrated) are inserted.

The endoscope connector 5 provided on an end portion of the universal cord 4 includes an electric connector portion 5a provided on a side face portion, and a light source connector portion 5b connected with a light source device which is an external device not illustrated.

Note that, to the electric connector portion 5a, a connector of an electric cable extended from a video processor which is an external device not illustrated is freely detachably connected. In addition, at the light source connector portion 5b, a light guide connector portion 5c housing the light guide bundle is disposed.

Next, the configuration of the bending operation lever portion 30 provided in the operation portion main body 14 will be described in more detail, based on Fig. 3.

The bending operation lever portion 30 provided on the back surface side of the operation portion main body 14 is configured by a lever of a so-called joystick type that can be tilted in all directions including the up-down/right-left directions, for example.

On a projecting end portion of the bending operation lever portion 30, as illustrated in Fig. 3, a finger contact portion 31 with which a thumb or the like of the user or the like can be brought into contact is provided. Note that the bending operation lever portion 30 is provided such that the user or the like can operate the finger contact portion 31 by the thumb of the hand grasping the grasping portion 13.

For the bending operation lever portion 30, a rod-like sliding body 37 in a roughly columnar shape which is a sliding portion is connected to a watertight holder portion 36 connected to the finger contact portion 31, and the sliding body 37 is housed so as to be freely movable back and forth along an axial (longitudinal axis) direction of a roughly cylindrical operation shaft 32 which is a lever shaft.

Note that, to the operation shaft 32 and the watertight holder portion 36 to which the sliding body 37 is connected, a tension spring 38, end portions of which are fixed to respectively formed outward flanges 32a and 36a, is interposed so as to extrapolate an upper part of the operation shaft 32, and the state in which the sliding body 37 is housed inside the operation shaft 32 is maintained by tension stress of the tension spring 38.

That is, the tension spring 38 configures a return member configured to impart the tensile stress to the sliding body 37 to return to the side of the operation portion main body 14 of the operation portion 2 along the axial (longitudinal axis) direction of the operation shaft 32.

Then, the bending operation lever portion 30 is provided with an exterior cover 33 as a waterproof boot for watertightly sealing a periphery of the watertight holder portion 36, the sliding body 37 and the operation shaft 32 and forming a sealed space inside the endoscope 1.

Note that, inside the operation portion 3, a bending operation mechanism not illustrated is connected to the operation shaft 32 of the bending operation lever portion 30. The bending operation lever portion 30 can bend the bending portion 7 in an arbitrary direction through a pulling action of each pulling wire by the bending operation mechanism.

The exterior cover 33 of the bending operation lever portion 30 is configured by a resin material which is an elastic member of soft rubber or the like, an inner peripheral edge portion of which close to the finger contact portion 31 watertightly covers an outer side of the operation shaft 32, and an outer peripheral edge portion of which is watertightly fixed to the operation portion main body 14 of the operation portion 3.

Specifically describing, for example, as illustrated in Fig. 3, the exterior cover 33 is configured by a doughnut-like member forming a meandering shape on a cross section, for which a mountain fold portion 33a and a valley fold portion 33b are formed at a middle portion from an outer peripheral side to an inner peripheral side.

For the exterior cover 33, seal rings 34 and 35 forming a thick bead shape are respectively and integrally formed at peripheral end portions on the inner peripheral side and the outer peripheral side.

Of the seal rings 34 and 35, the seal ring 34 on the inner peripheral side close to the finger contact portion 31 is brought into press contact with the finger contact portion 31 by the watertight holder portion 36, and thus watertightness is held from the finger contact portion 31 to the watertight holder portion 36, the sliding body 37 and the operation shaft 32.

On the other hand, of the seal rings 34 and 35, the seal ring 35 on the outer peripheral side on the side of the operation portion main body 14 is brought into press contact and fixed by a non-illustrated cover holder including a plurality of holder rings provided in the operation portion main body 14, and thus the watertightness is held.

In this way, the bending operation lever portion 30 projected from the operation portion main body 14 to be moved is provided in the state in which the watertightness is held from a position close to the finger contact portion 31 to the operation portion main body 14.

In the endoscope 1 of the present embodiment configured as above, a positive pressure is applied to the inside and pressurization is performed during a leak test for determining whether or not the watertightness is secured inside during cleaning after use or the like, and thus an internal pressure rises.

At the time, in the bending operation lever portion 30, as illustrated in Fig. 4, the sliding body 37 connected through the watertight holder portion 36 to the finger contact portion 31 is extended from the operation shaft 32 where the sliding body 37 is housed against the tensile stress of the tension spring 38, and the finger contact portion 31 is moved in a direction of separating from the operation portion main body 14 of the operation portion 3.

Therefore, in response to the rise of the internal pressure, the mountain fold portion 33a and the valley fold portion 33b are eliminated and the exterior cover 33 is deformed so as to be swollen in a so-called dome shape. That is, the exterior cover 33 is prevented from being folded even when the internal pressure rises during the leak test.

Note that, in the endoscope 1, the internal pressure is depressurized to a negative pressure after the leak test. At the time, by the tensile stress of the tension spring 38, the sliding body 37 is housed inside the operation shaft 32 and returned to the state during the use.

By such a configuration, in the endoscope 1, since a folded part of the exterior cover 33 is not generated even when automatic cleaning is performed by an endoscope cleaning apparatus configured to perform the leak test during a cleaning process, need of manually performing cleaning again is eliminated, and troublesome time and labor can be prevented.

Thus, the endoscope 1 of the present embodiment can be configured without the need of time and labor in cleaning by preventing the exterior cover 33 covering the outer side portion of the bending operation lever portion 30 from being folded in the state in which the positive pressure is applied to the inside during a leak test process, even when used in the endoscope cleaning apparatus configured to execute a series of processes from the leak test to the cleaning.

Note that, without being limited to execution of the cleaning in the endoscope cleaning apparatus configured to execute a series of processes from the leak test to the cleaning, of course the endoscope 1 may be manually cleaned, or may be cleaned using the endoscope cleaning apparatus which does not perform the leak test during a cleaning process.

Note that, even when the tensile stress of the tension spring 38 is imparted, since the sliding body 37 is freely movable back and forth to the operation shaft 32, the bending operation lever portion 30 described in the embodiment described above is structured such that the finger contact portion 31 is slid to be freely projected and sunk even during the use of the endoscope 1.

In this way, the endoscope 1 is not easy to use during the use when the finger contact portion 31 of the bending operation lever portion 30 is freely projected and sunk, it is preferable to include a structure of fixing the finger contact portion 31 during the use.

Therefore, as illustrated in following modifications, some lock mechanisms configured to restrict back and forth movement of the sliding body 37 and fix the sliding body 37 in the state of being moved to the side of the operation portion 3 such that the finger contact portion 31 of the bending operation lever portion 30 is not freely projected are illustrated.

### (First Modification)

Fig. 5 is a perspective view illustrating a configuration of a bending operation lever portion of the first modification.

As illustrated in Fig. 5, for the bending operation lever portion 30, a lock groove 32b which is an L-shaped groove portion is formed on the operation shaft 32, and a lock pin 36b to engage in the lock groove 32b is provided on the sliding body 37 connected through the watertight holder portion 36 to the finger contact portion 31.

In the bending operation lever portion 30, when the finger contact portion 31 is sunk, by inserting and removing the lock pin 36b to/from a part orthogonal to the operation shaft 32 of the lock groove 32b by turning around the axis of the sliding body 37, the state of locking the sliding body 37 and the state of unlocking the sliding body 37 in freely slidable state can be switched.

In this way, the bending operation lever portion 30 can be freely switched between the time of the use when the finger contact portion 31 is prevented from being freely projected, and the time of the cleaning during which the leak test or the like is performed when the finger contact portion 31 is slid and freely projected and sunk.

### (Second Modification)

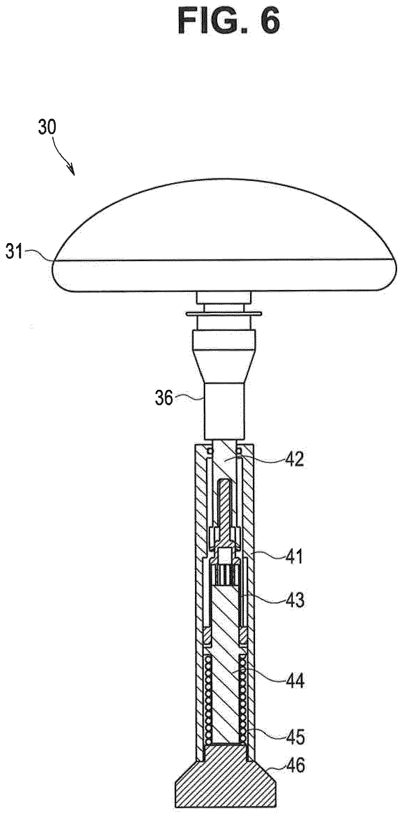

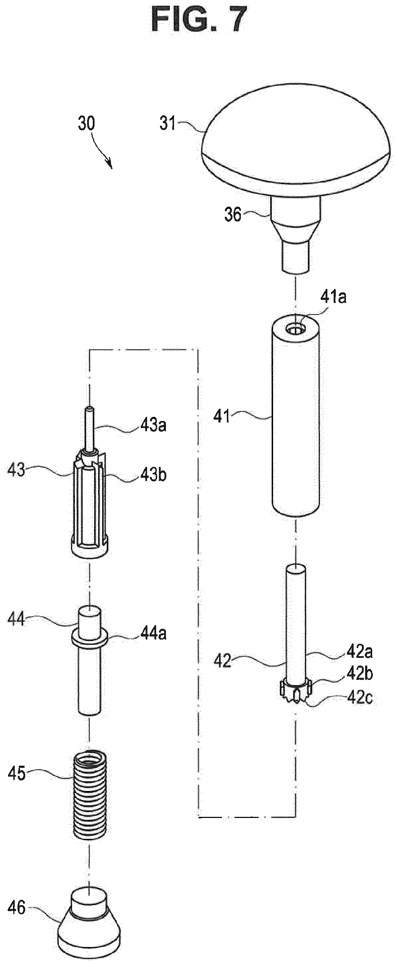

Fig. 6 is a partial sectional view illustrating a configuration of a bending operation lever portion of the second modification, and Fig. 7 is an exploded perspective view illustrating the configuration of the bending operation lever portion. Note that, in the drawings describing the bending operation lever portion 30 here, the exterior cover 33 is not illustrated and is omitted.

As illustrated in Fig. 6 and Fig. 7, the bending operation lever portion 30 is a so-called knock type delivery lock mechanism portion, and is configured including a roughly cylindrical exterior portion 41, a knock rod 42, a rotor 43, a spring retainer 44 in a roughly columnar shape, a coil spring 45, and a bearing 46.

The exterior portion 41 includes an opening portion 41a at one end, and a non-illustrated groove is formed on an inner peripheral surface. The knock rod 42 is housed inside the exterior portion 41 such that a rod-like operation rod body 42a is projected from the opening portion 41a of the exterior portion 41, and is operated so as to be projected and sunk to/from the exterior portion 41.

In addition, the knock rod 42 includes a plurality of projection portions 42b moved straight along the groove (not illustrated) of the exterior portion 41 at an outer peripheral portion, and a recessed and projected portion 32c is formed on a surface on an opposite side of the operation rod body 42a.

Note that the knock rod 42 is connected to the watertight holder portion 36 by being screwed or the like, and is connected to the finger contact portion 31 through the watertight holder portion 36.

The rotor 43 includes a rod body 43a that is inserted into the knock rod 42, turned and held, and a cam 43b for being in contact with the recessed and projected portion 42c and rotated according to projection/sinking of the knock rod 42 is formed at the outer peripheral portion. That is, the cam 43b is brought into contact with the recessed and projected portion 42c of the knock rod 42 and the rotor 43 is axially rotated.

For the spring retainer 44, an outward flange 44a is formed in the middle of the outer peripheral portion. The outward flange 44a is brought into contact with one end of the coil spring 45, and receives energizing force at one part. Note that the bearing 46 is connected to the opening portion of the exterior portion 41 by being screwed or the like.

That is, an operation shaft here is configured by the portions from the knock rod 42 projected from the exterior portion 41 and connected through the watertight holder portion 36 to the finger contact portion 31 to the bearing 46 closing the opening portion of the exterior portion 41.

In the bending operation lever portion 30 configured in this way, when the knock rod 42 is depressed by an operator, the rotor 43 is rotated in a predetermined direction, and the cam 43b is hooked to a projected portion formed on an inner surface of the exterior portion 41 and including a V-shaped end face not illustrated in accordance with the rotating position.

At the time, the rotor 43 pressurizes the spring retainer 44, and is restrained in the state of compressing the coil spring 45. Thus, the bending operation lever portion 30 is turned to the state in which a portion of the knock rod 42 is sunk in the exterior portion 41, and the finger contact portion 31 is turned to the state of being moved to the side of the operation portion main body 14. In the state, the endoscope 1 is ready to be used.

Then, in the bending operation lever portion 30, when the finger contact portion 31 is depressed by the operator from the state in which a portion of the knock rod 42 is sunk in the exterior portion 41, the rotor 43 is rotated further in the predetermined direction, and the cam 43b is detached from the V-shaped end face (not illustrated) formed at the projected portion of the exterior portion 41.

At the time, the rotor 43 receives the energizing force of the coil spring 45 through the spring retainer 44 and is returned, and the knock rod 42 is turned to the state of being projected from the exterior portion 41. Thus, the finger contact portion 31 is turned to the state of being moved in a direction of separating from the operation portion main body 14. In this state, the time of the cleaning during which the leak test or the like is performed for the endoscope 1 comes.

That is, the bending operation lever portion 30 includes a knock type delivery lock mechanism portion adopted in a knock type ball-point pen or the like, and is freely switched between the time of the use when the finger contact portion 31 is moved to the side of the operation portion main body 14 and the time of the cleaning during which the leak test or the like is performed when the finger contact portion 31 is moved in the direction of separating from the operation portion main body 14.

### (Third Modification)

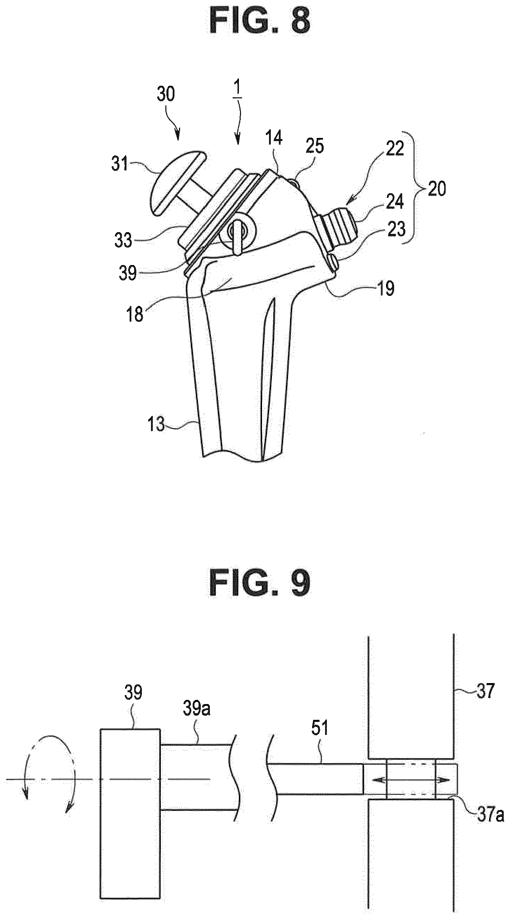

Fig. 8 is a right side view illustrating an appearance configuration of an operation portion of an endoscope of the third modification, and Fig. 9 is a conceptual diagram illustrating a structure configured to lock/unlock a sliding body by a lock lever.

The operation portion 3 of the endoscope 1 is provided with a lock lever 39 as illustrated in Fig. 8, and the sliding body is locked/unlocked by turning the lock lever as illustrated in Fig. 9.

Specifically, as illustrated in Fig. 9, when the lock lever 39 is turned, a lock pin 51 is moved back and forth in linkage with turning of a lock shaft 39a.

Then, a lock state in which the back and forth movement of the sliding body 37 to the operation shaft 32 not illustrated here is restricted by the lock pin 51 being fitted to a peripheral groove 37a formed on the sliding body 37, and an unlock state in which the sliding body 37 is freely movable back and forth to the operation shaft 32 by the lock pin 51 being detached from the peripheral groove 37a are switched.

In this way, the endoscope 1 is freely switched between the time of the use when the back and forth movement of the sliding body 37 is restricted by a turning operation of the lock lever 39 and the finger contact portion 31 is prevented from being freely projected, and the time of the cleaning during which the leak test or the like is performed when the sliding body 37 is freely movable back and forth, the finger contact portion 31 is slid and freely projected and sunk.

Note that the structure to fit the lock pin 51 in order to restrict the back and forth movement of the sliding body 37 is not limited to the peripheral groove 37a, and various shapes such as D cut or a hole portion may be applied.

### (Fourth Modification)

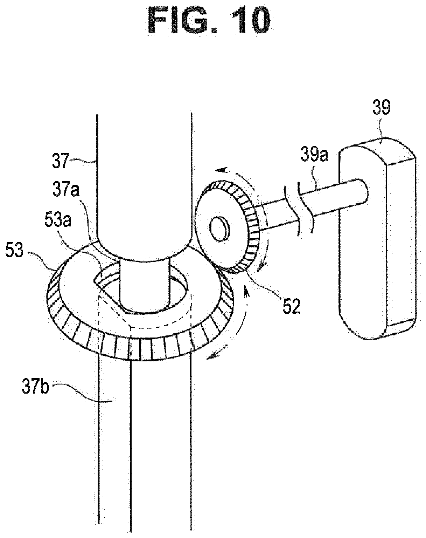

Fig. 10 is a perspective view illustrating a structure configured to lock/unlock a sliding body by a lock lever of the fourth modification. Note that, in the present modification, a different configuration of locking/unlocking the sliding body 37 by the turning operation of the lock lever 39 in the third modification is illustrated.

As illustrated in Fig. 10, a bevel gear 52 is provided on an end portion of the lock shaft 39a of the lock lever 39, and a lock plate 53 disposed so as to be roughly orthogonal to the bevel gear 52 and provided with a gear groove in a bevel gear shape formed to be engaged with the bevel gear 52 and turned is provided.

The lock plate 53 includes a hole portion 53a in a D cut shape formed at a center, and is disposed at the peripheral groove 37a of the sliding body 37. Note that the lock plate 53 is freely turnably held by a turning holding member not illustrated.

The sliding body 37 is in the D cut shape on the cross section with a plane 37b formed in the longitudinal axis direction of an outer peripheral portion to be a lower side here than the peripheral groove 57a. Note that the cross section D cut shape of the sliding body 37 and the D cut shape of the hole portion 53a of the lock plate 53 are similar shapes.

In the endoscope 1 configured in this way, when the lock lever 39 is turned, the lock plate 53 is turned in linkage with the turning of the bevel gear 52.

Then, at a turning position of the lock plate 53 where the D cut shapes of the hole portion 53a of the lock plate 53 and the sliding body 37 do not coincide, the lock plate 53 is brought into contact and hooked to the peripheral groove 37a formed on the sliding body 37, and thus the lock state of restricting the back and forth movement of the sliding body 37 to the operation shaft 32 not illustrated here is attained.

On the other hand, at the turning position of the lock plate 53 where the D cut shapes of the hole portion 53a of the lock plate 53 and the sliding body 37 coincide, the sliding body 37 is freely insertable through the hole portion 53a, and the unlock state in which the sliding body 37 is freely movable back and forth to the operation shaft 32 is attained.

Even in such a configuration, similarly to the third modification, the endoscope 1 is freely switched between the time of the use when the back and forth movement of the sliding body 37 is restricted by the turning operation of the lock lever 39 and the finger contact portion 31 is prevented from being freely projected, and the time of the cleaning during which the leak test or the like is performed when the sliding body 37 is freely movable back and forth, the finger contact portion 31 is slid and freely projected and sunk.

Note that the invention described in the above-described embodiment is not limited to the embodiment and the modifications and can be variously modified without departing from the scope in an implementation phase in addition. Further, the above-described embodiment includes the inventions in various stages, and various inventions can be extracted by appropriate combinations in a plurality of disclosed constituent elements.

For example, even when some constituent elements are deleted from the entire constituent elements indicated in the embodiment, in the case in which the described problem can be solved and the described effect can be obtained, the configuration from which the constituent elements are deleted can be extracted as the invention.

According to the present invention, it is possible to provide the endoscope that can prevent an exterior cover covering an outer side portion of an operation lever from being folded in the state in which a positive pressure is applied to the inside in a leak test process, does not need the time and labor of a manual leak test, and does not choose a model of an automatic endoscope cleaning apparatus, even when used in the endoscope cleaning apparatus configured to execute a series of processes from the leak test to the cleaning.

The present application is filed with Japanese Patent Application No. 2016-011465 filed in Japan on January 25, 2016 as a base of a claim of priority, and the above-described disclosure content is cited in the present description and scope of claims.

## Claims

1. An endoscope comprising:
an operation portion provided on a proximal end side of an insertion portion including a bending portion;
an operation lever portion provided in the operation portion, the operation lever portion including an operation shaft and being capable of adjusting a bending angle of the bending portion in linkage with a tilting action;
a sliding body disposed so as to be freely movable back and forth in an axial direction of the operation shaft;
a flexible exterior cover watertightly fixed to the operation portion so as to cover an outer periphery of the sliding body and the operation shaft, the flexible exterior cover being deformable by fluctuation of an internal pressure of the operation portion; and
a return member configured to return the sliding body to a side of the operation portion when the internal pressure of the operation portion is depressurized.

2. The endoscope according to claim 1, wherein the sliding body is housed so as to be freely movable back and forth in the operation shaft.

3. The endoscope according to claim 1 or claim 2, wherein the return member is a tension spring connected to the sliding body at one end.

4. The endoscope according to any one of claim 1 to claim 3, comprising a lock mechanism configured to restrict back and forth movement of the sliding body and fix a state in which the sliding body is moved to the side of the operation portion.
